# EUROPEAN PATENT APPLICATION

(11) **EP 2 813 224 A1**
(43) Date of publication of application: **17.12.2014**
(21) Application number: 13747280.9
(22) Date of filing: 06.02.2013
(51) Int. Cl.: A61K 31/198, A61P 21/00, A61P 29/00

(54) **PROPHYLACTIC OR THERAPEUTIC AGENT FOR IDIOPATHIC INFLAMMATORY MYOPATHIES**

(30) Priority: 06.02.2012 JP 2012023521
(71) Applicant: Ajinomoto Co., Inc., Tokyo, 104-8315 (JP); National University Corporation Tokyo Medical and Dental University, Bunkyo-ku Tokyo 113-8510 (JP)
(72) Inventor: KOHSAKA, Hitoshi, Tokyo 113-8510 (JP); TAKEHANA, Kenji, Kawasaki-shi Kanagawa 210-8681 (JP); TAGATA, Yusuke, Kawasaki-shi Kanagawa 210-8681 (JP)
(74) Representative: Nicholls, Kathryn Margaret
(86) International application number: PCT/JP2013/052730
(87) International publication number: WO 2013/118773

(57) **Abstract**

The present invention aims to provide a prophylactic or therapeutic agent for idiopathic inflammatory myopathy or idiopathic inflammatory myopathy associated with steroid-induced myopathy that develops during the course of treatment.

The present invention provides a prophylactic or therapeutic agent for idiopathic inflammatory myopathy or idiopathic inflammatory myopathy associated with steroid-induced myopathy that develops during the course of treatment, which contains isoleucine, leucine and valine as active ingredients.

## Description

### Technical Field

The present invention relates to a prophylactic or therapeutic agent for idiopathic inflammatory myopathy or idiopathic inflammatory myopathy associated with steroid-induced myopathy. More particularly, the present invention relates to a prophylactic or therapeutic agent for idiopathic inflammatory myopathy or idiopathic inflammatory myopathy associated with steroid-induced myopathy, which comprises isoleucine, leucine and valine as active ingredients.

### Background Art

Idiopathic inflammatory myopathy is a general term for myopathy classified according to the disease type classification of Wartmann and Olsen into polymyositis, dermatomyositis, dermatomyositis without muscular symptom, child dermatomyositis, myositis occurring in association with malignant tumor, myositis occurring in association with other collagen disease and inclusion body myositis. Pathological findings of infiltration of inflammatory cells into the muscle tissues, and non-uniform size of the muscle fibers, which is associated with muscle fiber necrosis and atrophy thereby, are acknowledged. Inflammation of muscle tissue leads to symptoms of systemic muscle weakness caused by muscle pain and muscle atrophy in some cases. Due to the weakness of the proximal muscles of the neck and limbs, daily performances of head elevation on awakening and going up and down the stairs, elevation of the upper limb and the like become difficult in some disease type, and aspiration pneumonia sometimes occurs due to dysphagia. Thus, muscle weakness in idiopathic inflammatory myopathy is a condition greatly affecting the QOL and prognosis of patients, which should be treated along with the calming of inflammations and prevention of complications.

At present, steroids are mainly used as the first-line drugs for the treatment of idiopathic inflammatory myopathy, and immunosuppressive agents are often used in combination. When a sufficient effect cannot be exhibited, a pulse therapy using a large amount of steroid is sometimes performed. Thus, therapies with a focus on the dosing of steroids are performed. These treatment methods are effective for calming inflammation of muscle tissues, but poor in the improvement of muscle atrophy and muscle weakness. Particularly, steroids show amyotrophic action as side effects, which may result in the symptoms of muscle weakness called steroid myopathy. Steroid myopathy is developed in patients using steroid drugs for a long time or in large quantities. To avoid steroid myopathy, there is no other way but to reduce the dose of steroid drug or cessation of the drug. However, reduction of the dose of steroid drug in the treatment of idiopathic inflammatory myopathy increases the risk of progression and recurrence of inflammation. Even if the reduction of drug was possible, the patient can only wait for the natural recovery of the muscle strength lost while moving the body in daily life, and complete recovery takes a long time. Therefore, the problem in idiopathic inflammatory myopathy is the absence of a therapeutic agent or a treatment method capable of simultaneously achieving the effects of anti-inflammation and improvement of muscle strength. Particularly, in idiopathic inflammatory myopathy associated with steroid-induced myopathy during the course of treatment, muscle weakness that continues after the calming of inflammation causes significant degradation of QOL. However, a method of preventing steroid-induced myopathy that develops during the course of treating idiopathic inflammatory myopathy or treating idiopathic inflammatory myopathy associated with steroid-induced myopathy is not known.

On the other hand, the possibility of valine in a branched chain amino acid (hereinafter to be also referred to as "BCAA") composition enhancing the effect of steroid drug and immunosuppressive agents in the treatment or prevention of rheumatoid arthritis, which is one of the inflammatory diseases, has been suggested (patent document 1). In the document, however, the efficacy against swelling of the limb was merely evaluated as a joint score, and the presence or absence of an anti-inflammatory effect of valine itself was not directly shown. Moreover, there is no description on the effectiveness of valine for idiopathic inflammatory myopathy. While the action of branched-chain amino acid on the muscles has been reported, it was mainly the improvement of muscle fatigue (patent document 2), are the usefulness of itself for idiopathic inflammatory myopathy is not known. Furthermore, branched-chain amino acid is known to inhibit steroid-induced myopathy and improve muscle strength, namely, to be useful for the prevention or treatment of steroid myopathy (patent document 3, non-patent document 3). However, usefulness of itself for idiopathic inflammatory myopathy and idiopathic inflammatory myopathy associated with steroid-induced myopathy is not known.

In addition, it has been shown by clinical studies in recent years that the drugs considered to have an anti-inflammatory effect on inflammatory diseases including rheumatoid arthritis are not necessarily effective for suppressing inflammation in idiopathic inflammatory myopathy, and show poor effect on the improvement of muscle strength. For example, in the tests including administration of Infliximab, which is an anti-TNF antibody effective for rheumatoid arthritis, to patients with polymyositis, dermatomyositis and inclusion body myositis, aggravation of inflammation and activation of type I interferons were observed (non-patent document 1). Also, an anti-inflammatory effect of Anakinra, which is an IL-1 receptor antagonist, on polymyositis and dermatomyositis has not been observed (non-patent document 2). As the situation stands, a drug effective for the treatment of idiopathic inflammatory myopathy or idiopathic inflammatory myopathy associated with steroid-induced myopathy that develops during the course of treatment has not been obtained heretofore.

### [Document List]

### [patent documents]

patent document 1: WO 2005/055997
patent document 2: JP-A-8-198748
patent document 3: WO 2008/072663

### [non-patent documents]

non-patent document 1: Ann Rheum Dis. 2008 Dec;67(12):1670-7
non-patent document 2: Ann Rheum Dis. 2011;70:A80-A81
non-patent document 3: Cell Metabolism 2010;13:170-182

### SUMMARY OF THE INVENTION

### Problems to be Solved by the Invention

The present invention aims to provide a prophylactic or therapeutic agent for idiopathic inflammatory myopathy or idiopathic inflammatory myopathy associated with steroid-induced myopathy that develop during the treatment. Means of Solving the Problems

The present inventors have conducted intensive studies in an attempt to solve the aforementioned problems and found that three kinds of branched chain amino acids of isoleucine, leucine and valine are effective for the prophylaxis or treatment of idiopathic inflammatory myopathy and idiopathic inflammatory myopathy associated with steroid-induced myopathy, particularly, suppress (soothe) inflammation of muscular tissue in idiopathic inflammatory myopathy and idiopathic inflammatory myopathy associated with steroid-induced myopathy, and further have an action to suppress symptoms of muscular atrophy to improve muscle strength. Based on these findings, the present inventors have conducted further studies and completed the present invention.

Accordingly, the present invention is as described below.
[1] A prophylactic or therapeutic agent for idiopathic inflammatory myopathy, comprising isoleucine, leucine and valine as active ingredients;
[2] the agent of [1], wherein the idiopathic inflammatory myopathy is selected from the group consisting of polymyositis, dermatomyositis, dermatomyositis without muscular symptom, child dermatomyositis, myositis occurring in association with malignant tumor, myositis occurring in association with other collagen disease, and inclusion body myositis;
[3] the agent of [1] or [2], which suppresses inflammation of muscular tissue;
[4] the agent of any of [1] - [3], which improves muscular atrophy and/or muscle weakness;
[5] the agent of any of [1] - [4], wherein the weight ratio of isoleucine, leucine and valine is 1:1 - 3:0.5 - 2.0;
[6] the agent of any of [1] - [5], whose daily dose for human is 3 - 90 g as a total amount of isoleucine, leucine and valine;
[7] a pharmaceutical composition comprising the agent of any of [1] - [6], and a pharmaceutically acceptable carrier;
[8] a prophylactic or therapeutic agent for idiopathic inflammatory myopathy, consisting of isoleucine, leucine and valine;
[9] a pharmaceutical composition consisting of the agent of [8], and a pharmaceutically acceptable carrier;
[10] a method of preventing or improving idiopathic inflammatory myopathy, comprising administering a composition containing isoleucine, leucine and valine as active ingredients to a subject of administration;
[11] the method of [10] for suppressing inflammation of muscular tissue, which comprises administering a composition containing isoleucine, leucine and valine as active ingredients to a subject of administration;
[12] the method of [10] for improving muscular atrophy and/or muscle weakness, which comprises administering a composition containing isoleucine, leucine and valine as active ingredients to a subject of administration;
[13] the method of any of [10] - [12], wherein the weight ratio of isoleucine, leucine and valine is 1:1 - 3:0.5 - 2.0;
[14] the method of any of [10] - [13], wherein the daily dose for human is 3 - 90 g as a total amount of isoleucine, leucine and valine;
[15] the agent of any of [1] - [6], wherein the idiopathic inflammatory myopathy is idiopathic inflammatory myopathy associated with steroid-induced myopathy which develops in the course of treatment;
[16] a pharmaceutical composition comprising the agent of [15], and a pharmaceutically acceptable carrier;
[17] the agent of [8], wherein the idiopathic inflammatory myopathy is idiopathic inflammatory myopathy associated with steroid-induced myopathy which develops in the course of treatment;
[18] a pharmaceutical composition comprising the agent of [17], and a pharmaceutically acceptable carrier;
[19] the method of any of [10] - [14], wherein the idiopathic inflammatory myopathy is idiopathic inflammatory myopathy associated with steroid-induced myopathy which develops in the course of treatment.

### Effect of the Invention

The agent or composition provided by the present invention, which contains isoleucine, leucine and valine as active ingredients, is particularly superior in the both effects of suppression of muscular tissue inflammation and improvement of muscle strength in idiopathic inflammatory myopathy and idiopathic inflammatory myopathy associated with steroid-induced myopathy. Therefore, it can prevent or treat the diseases more effectively by the administration to patients with idiopathic inflammatory myopathy or idiopathic inflammatory myopathy associated with steroid-induced myopathy.

In the treatment of idiopathic inflammatory myopathy, steroid drugs have conventionally been used from the aspect of anti-inflammation and, when the effect thereof is insufficient, an immunosuppressant is used in combination. However, both medicaments do not have an effective muscle strength improving action. Therefore, simultaneous achievement of the inflammation suppressive action on muscular tissues and the muscle strength improving effect are effects specific to the agent of the present invention. Moreover, effective muscle strength improvement effect without reduction of the dose and cessation of the steroid drug in idiopathic inflammatory myopathy associated with steroid-induced myopathy is an effect specific to the agent of the present invention.

Since the three kinds of branched chain amino acids of isoleucine, leucine and valine are substances having established safety, the prophylactic or therapeutic agent of the present invention for idiopathic inflammatory myopathy and idiopathic inflammatory myopathy associated with steroid-induced myopathy can be provided as a highly safe drug with scarce side effects.

### Brief Description of the Drawings

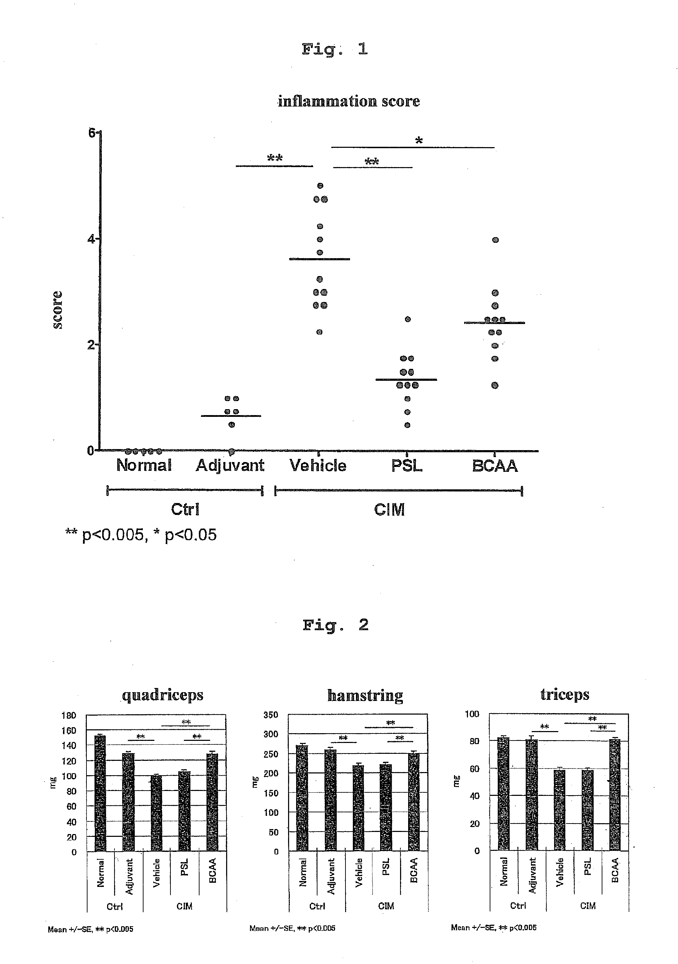

Fig. 1 is a graph showing comparison of inflammation scores of C-protein induction type mouse myositis model (CIM mouse) among BCAA administration group, prednisolone (PSL) administration group and vehicle administration group, and normal group and adjuvant immunization group of control (Ctrl) mouse, wherein the vertical axis shows inflammation scores of the mice.
Fig. 2 includes graphs showing comparison of muscle weight of quadriceps, hamstring and triceps of CIM mouse among BCAA administration group, PSL administration group and vehicle administration group, and normal group and adjuvant immunization group of Ctrl mouse, wherein the vertical axis shows the weight (mg) of various muscles.

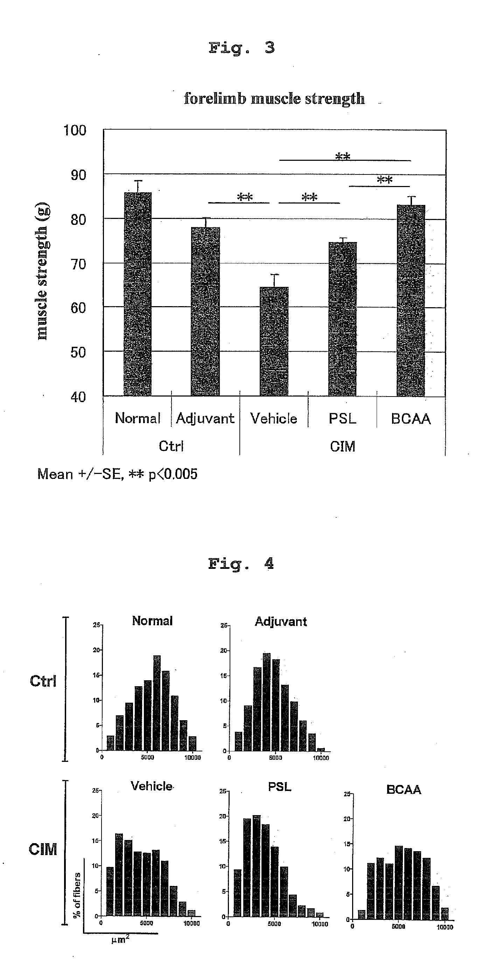

Fig. 3 is a graph showing comparison of forelimb muscle strength measurement values of CIM mouse among BCAA administration group, PSL administration group and vehicle administration group, and normal group and adjuvant immunization group of Ctrl mouse, wherein the vertical axis shows the measurement values (g) of forelimb muscle strength of the mice.
Fig. 4 includes graphs showing comparison of the distribution of fast muscle fiber sectional areas of CIM mouse among BCAA administration group, PSL administration group and vehicle administration group, and normal group and adjuvant immunization group of Ctrl mouse, wherein the horizontal axis shows the area (µm²) of fast muscle fiber and the vertical axis shows the proportion (%) of muscle fiber.

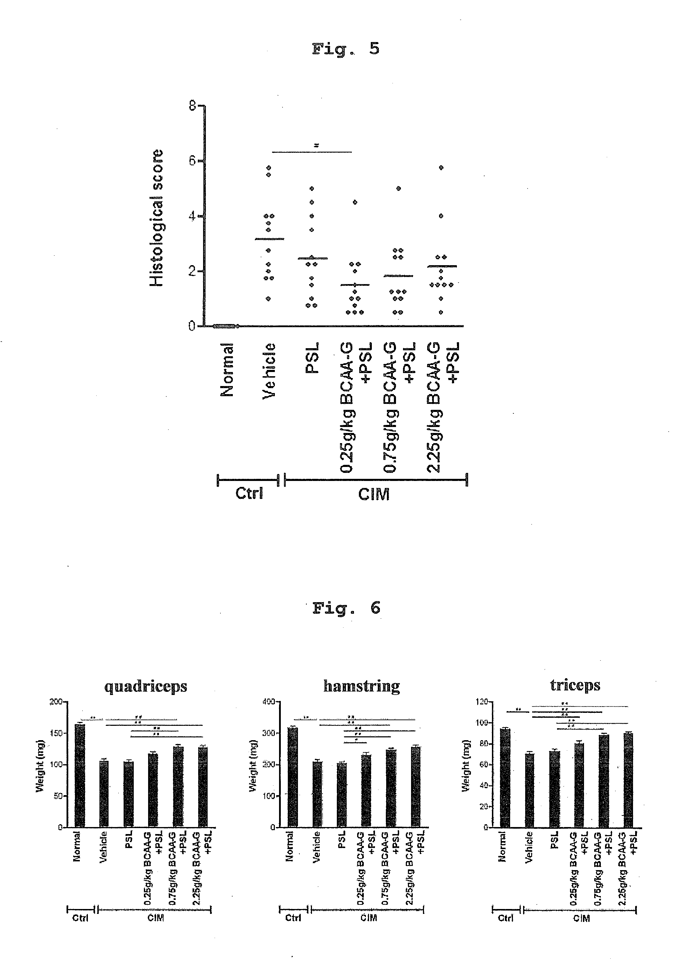

Fig. 5 is a graph showing comparison of inflammation scores of CIM mouse among BCAA+PSL combined administration group and PSL single administration group, and normal group and vehicle administration group of Ctrl mouse, wherein the vertical axis shows inflammation scores of the mice.
Fig. 6 includes graphs showing comparison of muscle weight of quadriceps, hamstring and triceps of CIM mouse among BCAA+PSL combined administration group, PSL single administration group and vehicle administration group, and normal group of Ctrl mouse, wherein the vertical axis shows the weight (mg) of various muscles.

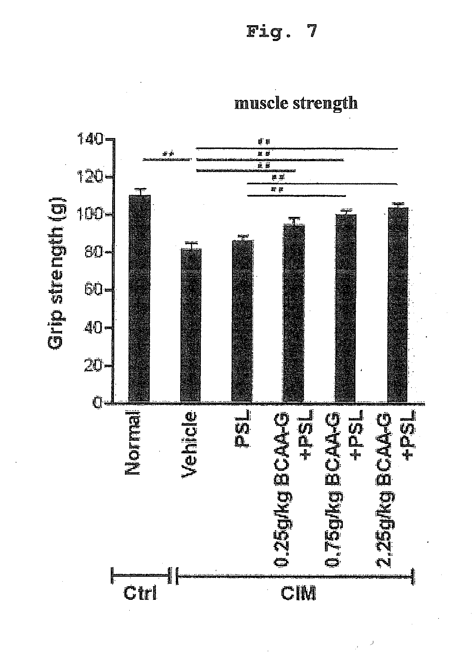

Fig. 7 is a graph showing comparison of forelimb muscle strength measurement values of CIM mouse among BCAA+PSL combined administration group, PSL single administration group and vehicle administration group, and normal group of Ctrl mouse, wherein the vertical axis shows the measurement values (g) of forelimb muscle strength of the mice.

### Description of Embodiments

The present invention provides a prophylactic or therapeutic agent for idiopathic inflammatory myopathy and idiopathic inflammatory myopathy associated with steroid-induced myopathy, which contains isoleucine, leucine and valine as active ingredients (in the present specification, sometimes to be referred to as "the agent of the present invention").

In the present invention, idiopathic inflammatory myopathy refers to a disease wherein infiltration of mononuclear cells is observed in the skeletal muscle of the four limbs or the trunk, which shows muscle disorders such as inflammation, degeneration and the like. As the symptoms of idiopathic inflammatory myopathy, inflammation of muscular tissue and muscle weakness are mainly observed as described above, and also, skin symptoms (e.g., heliotrope rash, Gottron's sign etc.), arthropathy (e.g., arthralgia, arthritis etc.), Raynaud's phenomenon, respiratory symptoms (e.g., interstitial pneumonia etc.), cardiac symptoms (e.g., arrhythmia, cardiac failure etc.), systemic symptoms (e.g., fever, general malaise etc.) and the like are observed.

The agent of the present invention also has a muscular tissue inflammation suppressive action and a muscle strength improving action, and may show, together with these actions or via these actions, an action to improve the above-mentioned other symptoms. When used in the present specification, the "prophylaxis" of idiopathic inflammatory myopathy and idiopathic inflammatory myopathy associated with steroid-induced myopathy means prevention of exteriorization of the above-mentioned symptoms of idiopathic inflammatory myopathy or steroid-induced myopathy in individuals who do not show such symptoms (including prevention of recurrence), and "treatment" means mitigation of, or prevention of the aggravation of, or delaying the symptoms of idiopathic inflammatory myopathy and idiopathic inflammatory myopathy associated with steroid-induced myopathy in individuals showing such symptoms.

Idiopathic inflammatory myopathy can be classified in more detail according to the above-mentioned clinical symptoms and the findings of the needle electromyography and the like. Examples of the classified idiopathic inflammatory myopathy include polymyositis, dermatomyositis, child dermatomyositis, myositis occurring in association with malignant tumor, myositis occurring in association with other collagen diseases (e.g., SLE (systemic lupus erythematosus), scleroderma etc.), inclusion body myositis and the like. In clinical findings, many patients are diagnosed with polymyositis, dermatomyositis or inclusion body myositis, among which many patients are diagnosed with polymyositis or dermatomyositis.

Therefore, the agent of the present invention is useful for all of the above-mentioned diseases (myositis). Particularly, it is effective for polymyositis and dermatomyositis, and most effective for polymyositis.

In recent years, moreover, a disease concept of dermatomyositis without muscular symptom has been accepted in idiopathic inflammatory myopathy. In such disease, skin symptoms characteristic of dermatomyositis are observed, but the muscle symptoms of inflammation of muscle tissue and muscle weakness are milder than other myositis, or such muscle symptoms are not observed at all.

The agent of the present invention is also useful for dermatomyositis without muscular symptoms, since it further improves mild muscular symptoms or prevents muscular symptoms that may be developed in the future.

While the onset mechanism of polymyositis and dermatomyositis has not been completely elucidated, muscular tissues in these diseases generally show infiltration of mononuclear cells into the periphery of non-necrotic skeletal muscle fibers is observed. The infiltrating inflammatory cells are, for example, T lymphocyte, B lymphocyte, NK cell, macrophage, dendritic cell and the like.

In polymyositis, it is considered that CD8 positive T cells infiltrate into the endomysium to cause muscle disorders, via perforin and granzyme B, of the muscle fibers expressing MHC-I on the surface of the cell membrane (Curr. Opin. Pharmacol., 10:346-352, 2010). On the other hand, it is suggested that activation of the complement cascade is involved in dermatomyositis, and the membrane attack complex formed is deposited on the vascular endothelium in endomysium, which develops vascular wall disorders and muscle ischemia. It is also considered that muscular atrophy in the periphery of muscle bundles results from such reduced blood flow in the muscle (Curr. Opin. Pharmacol., 10:346-352, 2010).

Since the agent of the present invention is useful for both polymyositis and dermatomyositis, three kinds of branched chain amino acids of isoleucine, leucine and valine can have an action to directly or indirectly act on the molecules involved in the action mechanism mentioned above and promote or suppress the activity thereof.

In one embodiment, isoleucine, leucine and valine can suppress of infiltration of inflammatory cells into muscular tissues.

Whether inflammatory cell has infiltrated can be examined by generating sections of muscular tissues collected by biopsy and the like, performing hematoxylin and eosin stain (HE stain) and observing the muscle sections. When the amount of inflammatory cells observed by HE stain decreases from before the administration of branched chain amino acids, it is concluded that infiltration of inflammatory cells has been suppressed.

In another embodiment, isoleucine, leucine and valine can improve muscular atrophy and/or muscle weakness.

Whether muscular atrophy has been improved can be examined by observing the changes in the condition of muscle fibers. As for the condition of muscle fibers, the muscle sections prepared in the same manner as above and stained in Elastica-van Gieson can be microscopically observed. When the amount of the stained muscle fibers (e.g., area in microscopic images) increases from before the administration of branched chain amino acids, it is concluded that the muscle fibers have been improved. The muscle fibers can also be stained with, besides the above-mentioned staining agents, antibodies against myosin heavy chain (MHC), laminin and the like, which are constituent proteins of muscle fibers. Other than the observation by staining, improvement of muscular atrophy can also be examined by collecting muscle cells from muscular tissues, and measuring the expression level of muscular atrophy-related genes (e.g., atrogon-1, MuRF-1 etc.) in the cells. When the gene is atrogon-1 or MuRF-1 and the expression level thereof decreases from before the administration of branched chain amino acids, it is concluded that the muscle fibers have been improved.

Whether muscle weakness has been improved can be examined using a commercially available muscle strength measurement device such as dynamometer and the like. When the muscle strength increases from before the administration of branched chain amino acids, it is concluded that the muscle weakness has been improved.

In polymyositis and dermatomyositis, inflammation in muscle tissue and muscle weakness are seen as symptoms. However, the degree of infiltration of inflammatory cells into the muscle and the severity of clinical symptoms of muscle weakness do not necessarily correlate with each other.

Therefore, the muscular tissue inflammation suppressive action and the muscle strength improving action of the agent of the present invention can be independent and separate actions.

Isoleucine, leucine and valine, which are contained as active ingredients in the agent of the present invention, may be usable in the form of any of L-form, D-form and DL-form. Preferred is L-form or DL-form, and more preferred is L-form. Isoleucine, leucine and valine obtained by, for example, hydrolysis of animal- or plant-derived natural protein can be used, or those obtained by a fermentation method or chemical synthesis method can be used.

Isoleucine, leucine and/or valine may be each used in the form of not only a free form but also a salt form. Examples of the salt form include acid addition salt, base addition salt and the like; however, any form can be taken as long as it is a chemically acceptable salt. Since the agent of the present invention is generally used for medical purposes, the salt form is preferably a pharmaceutically acceptable salt.

Examples of the pharmaceutically acceptable salt include salts with acid and salts with base. Examples of the acid to be added to isoleucine, leucine or valine to form a pharmaceutically acceptable salt include inorganic acids such as hydrogen chloride, hydrobromic acid, sulfuric acid, phosphoric acid and the like, organic acids such as acetic acid, lactic acid, citric acid, tartaric acid, maleic acid, fumaric acid, monomethylsulfuric acid and the like. Examples of the base to be added to isoleucine, leucine or valine to form a pharmaceutically acceptable salt include metal hydroxide such as sodium, potassium and the like, metal carbonates such as calcium and the like, inorganic bases such as ammonia and the like, and organic bases such as ethylenediamine, propylenediamine, ethanolamine, monoalkylethanolamine, dialkylethanolamine, diethanolamine, triethanolamine and the like.

The mixing ratio (weight ratio) of isoleucine, leucine and valine contained in the agent of the present invention can be appropriately adjusted to fall within the range where the agent of the present invention shows a desired activity or a prophylactic or therapeutic effect for idiopathic inflammatory myopathy or idiopathic inflammatory myopathy associated with steroid-induced myopathy. For example, the mixing ratio of the three kinds of branched chain amino acids (isoleucine:leucine:valine) in a weight ratio is generally 1:1 - 3:0.5 - 2.0, preferably 1:1.5 - 2.5:0.8 - 1.5, more preferably 1:1.9 - 2.2:1.1 - 1.3, most preferably 1:2:1.2. When the agent of the present invention contains a salt of isoleucine, a salt of leucine or a salt of valine, the weight ratio is calculated by converting the salt of each branched chain amino acid to a free form. When the weight ratio of isoleucine, leucine and valine is within the above-mentioned range, an effective prophylactic or therapeutic effect for idiopathic inflammatory myopathy and idiopathic inflammatory myopathy associated with steroid-induced myopathy can be obtained.

The agent of the present invention is useful as a medicament, and the application target thereof includes mammals (e.g., human, mouse, rat, hamster, rabbit, cat, dog, bovine, sheep, monkey etc.). The application target of the agent of present invention is preferably human. For application to a mammal other than human, the amount of intake of the agent of the present invention can be appropriately controlled according to the body weight or size of the animal.

The administration method for using the agent of the present invention as a medicament may be any of oral administration and parenteral administration. Examples of the dosage form of the oral administration include solid agents such as powder, granule, capsule, tablet, chewable and the like, and liquids such as solution, syrup and the like. Examples of the dosage form of the parenteral administration include injection, transfusion, transnasal spray, transpulmonary spray and the like.

To reduce the burden on patients, the branched chain amino acids of isoleucine, leucine and valine are preferably administered orally to the target. On the other hand, when oral administration is difficult for the patient, it is transvenously or intra-arterially administered by infusion.

In addition, the agent of the present invention can be formulated as a pharmaceutical composition by blending, when necessary for formulation, an appropriate pharmaceutically acceptable carrier such as excipient, binder, lubricant, solvent, disintegrant, solubilizing agent, suspending agent, emulsifier, isotonic agent, stabilizer, soothing agent, preservative, antioxidant, correctives, colorant and the like (hereinafter sometimes to be referred to as "the composition of the present invention"). The agent of the present invention can be formulated into the above-mentioned dosage forms by a conventional method. In the embodiment of the composition of the present invention, the respective active ingredients of isoleucine, leucine and valine are preferably contained in a single composition since administration is convenient; however, the three kinds of branched chain amino acids each singly or in any combination may be contained in plural compositions.

Examples of the excipient include organic excipients such as saccharides (lactose, glucose, D-mannitol and the like), starches, celluloses (crystalline cellulose and the like) and the like, inorganic excipients such as calcium carbonate, kaolin, etc. and the like. Examples of the binder include pregelatinized starch, gelatin, gum arabic, methylcellulose, carboxymethylcellulose, sodium carboxymethylcellulose, crystalline cellulose, D-mannitol, trehalose, hydroxypropylcellulose, hydroxypropylmethylcellulose, polyvinylpyrrolidone, polyvinyl alcohol and the like. Examples of the lubricant include fatty acid salts such as stearic acid, stearic acid salt and the like, talc, silicates and the like. Examples of the solvent include purified water, physiological saline and the like. Examples of the disintegrant include low-substituted hydroxypropylcellulose, chemically modified cellulose, starches and the like. Examples of the solubilizing agent include polyethylene glycol, propylene glycol, trehalose, benzyl benzoate, ethanol, sodium carbonate, sodium citrate, sodium salicylate, sodium acetate and the like. Examples of the suspending agent or emulsifier include sodium lauryl sulfate, gum arabic, gelatin, lecithin, glycerol monostearate, polyvinyl alcohol, polyvinylpyrrolidone, celluloses such as sodium carboxymethylcellulose and the like, polysorbates, polyoxyethylene hydrogenated castor oil and the like. Examples of the isotonic agent include sodium chloride, potassium chloride, saccharides, glycerol, urea and the like. Examples of the stabilizer include polyethylene glycol, dextran sodium sulfate, other amino acids and the like. Examples of the soothing agent include glucose, calcium gluconate, procaine hydrochloride and the like. Examples of the preservative include paraoxybenzoates, chlorobutanol, benzyl alcohol, phenethyl alcohol, dehydroacetic acid, sorbic acid and the like. Examples of the antioxidant include sulfite, ascorbic acid and the like. Examples of the correctives include sweetener, flavor and the like, which are generally used in the pharmaceutical field. Examples of the colorant include those generally used in the pharmaceutical field.

The content of the branched chain amino acids (isoleucine, leucine and valine) contained in the composition of the present invention can be appropriately determined according to the form of the preparation and the like. For example, when the composition of the present invention is a jelly, the content of the branched chain amino acids is generally 5 - 50 wt%, preferably 10 - 30 wt%, relative to the whole composition. When the composition of the present invention is granule, the content of the branched chain amino acids is generally 50 - 100 wt%, preferably 90 - 100 wt%, relative to the whole composition. Here, the "content" is the total weight ratio of the three kinds of branched chain amino acids relative to the weight of the composition of the present invention. Therefore, for example, when the respective active ingredients of isoleucine, leucine and valine are contained in a single composition, the content thereof is the ratio of the total weight of the three kinds of branched chain amino acids relative to the weight of the composition. When respective active ingredients are contained singly, or in any combination in plural compositions, the content thereof is the ratio of the total weight of the three kinds of branched chain amino acids relative to the total weight of the respective compositions. The above-mentioned "weight ratio" is a ratio of the weight of the respective branched chain amino acids contained in the composition of the present invention. For example, when the respective active ingredients of isoleucine, leucine and valine are contained in a single composition, it is a ratio of the content of individual branched chain amino acids, and when each of the active ingredients is contained singly or in any combination in plural compositions, it is a ratio of the weight of each active ingredient contained in each composition.

Preferable examples of the agent or composition of the present invention include a branched chain amino acid preparation, LIVACT (registered trade mark) granule (Ajinomoto Co., Inc.) (agent for oral administration) containing isoleucine, leucine and valine at a weight ratio of 1:2:1.2 (isoleucine: 0.952 g, leucine: 1.904 g, valine: 1.144 g). In addition, preferable examples of an agent for parenteral administration include high concentration amino acid infusions, AMINIC ((registered trade mark) intravenous drip injection (Ajinomoto Pharmaceutical Co., Inc.)), and Morihepamin ((registered trade mark) intravenous drip injection (Ajinomoto Pharmaceutical Co., Inc.)).

While the dose (amount to be ingested) of the agent or composition of the present invention to human varies depending on the age, body weight, pathology of target patient, administration method and the like, it is generally isoleucine 1 - 30 g, leucine 1 - 30 g, and valine 1 - 30 g per day for one person. The dose for general adult is preferably isoleucine 2 - 10 g, leucine 4 - 20 g, and valine 2 - 10 g, more preferably isoleucine 2.5 - 3.0 g, leucine 5.0 - 7.0 g, and valine 3.0 - 4.0 g, per day for one person. In addition, the dose as the total amount of the three kinds of the branched chain amino acids is generally about 3 - 90 g, preferably about 3 - 20 g, per day for an adult, which is administered in 1 - 5, preferably 2 - 4, portions divided as necessary. When the agent or composition of the present invention contains salts of the branched chain amino acids, the dose is calculated by converting the salt of each branched chain amino acid to a free form. The timing of the administration is not particularly limited and may be, for example, before meal, between meals or after meal. Also, the dosing period is not particularly limited.

In calculation of the dose (amount of intake) of the branched chain amino acids, which are the active ingredients used in the present invention, since the range of the amount of the active ingredients of the medicament to be used for the purpose of treatment, prophylaxis and the like of target diseases in the present invention is determined by the aforementioned calculation method, when branched chain amino acids are ingested or administered for different purposes, for example, to satisfy the need in ordinary eating habits or treatment of other diseases, such branched chain amino acids do not need to be included in the aforementioned calculation. For example, the daily amount of branched chain amino acids to be ingested from ordinary eating habits does not need to be excluded from the calculation of the aforementioned daily dose of the active ingredients in the present invention.

In the present invention, the ratio of actual dose is a ratio of a single dose or a daily dose of each active ingredient per one subject of administration (i.e., patient). For example, when each active ingredient of isoleucine, leucine and valine is contained in a single preparation and administered to a subject of administration, the weight ratio corresponds to the dose ratio. When each active ingredient is contained separately or in any combination thereof in plural preparations and administered, the dose ratio corresponds to a ratio of the total amount of each active ingredient in each preparation administered at one time or in one day.

Isoleucine, leucine and valine have already been widely used in the fields of medicaments and foods, and the safety has been established. For example, the acute toxicity (LD₅₀) of the agent and composition of the present invention containing these branched chain amino acids at a ratio of 1:2:1.2 is not less than 10 g/kg by oral administration to mouse.

As mentioned above, the agent or composition of the present invention is useful for the prophylaxis or treatment of idiopathic inflammatory myopathy and idiopathic inflammatory myopathy associated with steroid-induced myopathy, and can also be used in combination with a conventionally-used prophylactic or therapeutic agent for idiopathic inflammatory myopathy. Here, being "used in combination" means use before, simultaneously with or after administration of a conventionally-used prophylactic or therapeutic agent for idiopathic inflammatory myopathy, including use as a blend mixing the both.

While the above-mentioned prophylactic or therapeutic agent for idiopathic inflammatory myopathy that can be used in combination with the agent or composition of the present invention is not particularly limited, for example, steroid drug, immunosuppressant and the like can be mentioned. Examples of the steroid drug include prednisolone, methylprednisolone, hydrocortisone, cortisone acetate, dexamethasone, triamcinolone, betamethasone and the like. Examples of the immunosuppressant include azathiopurine, methotrexate, cyclosporine, tacrolimus, mycophenol acid, various antibody medicaments, gamma globulin and the like. One kind alone of these medicaments may be combined with the agent or composition of the present invention, or two or more kinds may be used in combination. The dose, dosing period, and administration interval of the medicament to be used in combination with the agent or composition of the present invention can be appropriately determined according to the conditions of the disease, target patients and the like.

As another embodiment of the present invention, the agent or composition of the present invention can enhance the effects of steroid drugs and immunosuppressants in the prophylaxis or treatment of idiopathic inflammatory myopathy. Thus, it is useful since the treatment effect can be enhanced and the side effects can be reduced than when a steroid drug or an immunosuppressant is used singly. Examples of the steroid drug and immunosuppressant include the above-mentioned medicaments, and steroid drugs (e.g., prednisolone) are preferable. The dose, dosing period, and administration interval of the medicament to be used in combination with the agent or composition of the present invention can be appropriately determined according to the conditions of the disease, target patients and the like.

Steroid drugs are often used as a drug of first alternative in the treatment of idiopathic inflammatory myopathy. When muscle weakness is observed during the course of treatment, it is difficult to determine whether it was caused by the aggravation of the primary disease or results from the complication with steroid-induced myopathy. When used in combination from the start of the steroid treatment of idiopathic inflammatory myopathy, the agent or composition of the present invention improves both inflammation and muscle weakness caused by the primary disease of idiopathic inflammatory myopathy. On the other hand, sufficient improvement of muscle strength cannot be obtained by a steroid treatment alone in many cases due to the complication with steroid-induced myopathy, and reduction of the dosing amount of the steroid drug sometimes cannot be avoided for the treatment aiming to improve muscle strength. Using the agent or composition of the present invention, the treatment can be continued without reduction of the dosing amount of the steroid drug during the course of treatment due to the complication with steroid-induced myopathy, and the muscle strength can be improved sufficiently.

### Examples

The present invention is described in more detail by referring to Examples. The following Examples specifically explain one embodiment of the present invention, and do not limit the present invention thereto.

### Example 1 Muscular tissue inflammation suppressive effect of BCAA in C-protein-induced mouse myositis model

Using C-protein-induced mouse myositis model (C-protein-induced Myositis: CIM), the effect of BCAA on the inflammation of muscular tissues was examined. For antigen immunization of C57BL/6 mouse (female, 8-week-old), an adjuvant containing 200 µg C-protein and 100 µg killed tuberculosis bacterium (Mycobacterium butyricum), and Complete Freund's adjuvant (CFA) as a component was intradermally injected to the hindpaw sole and tail base, C-protein-free CFA injected to the base of forelimb, and 0.25 µg pertussis toxin was intraperitoneally injected. As an adjuvant control without immunization with an antigen, CFA was intradermally injected to the same sites. From three days after immunization, 0.75 g/kg BCAA (mixture of isoleucine, leucine and valine at a weight ratio of isoleucine:leucine:valine=1:2:1.2) or 20 mg/kg Prednisolone (PSL) was orally administered consecutively, and they were used as BCAA administration group and PSL administration group. In addition, 0.5% methylcellulose and 5% gamma cyclodextrin were orally administered instead of BCAA and the like to give Vehicle administration group. After 21 days from immunization, the muscle was collected, and the levels of inflammation of quadriceps and flexor muscles were evaluated by the inflammation score. The inflammation score is an average score of quadriceps and flexor muscles, which was determined by scoring the number of muscle fibers in the region where infiltration of inflammatory cells and muscle fiber necrosis were observed in the HE stained muscle section according to the following criteria.

Scoring method: Grade 0 = no infiltration, Grade 1 = 1 fiber, Grade 2 = 2-5 fibers, Grade 3 = 6-15 fibers, Grade 4 = 16-30 fibers, Grade 5 = 31-100 fibers, Grade 6 = 101 fibers or more. When infiltration of the same score was found in plural sites of the same muscle, 0.5 point was added to the score.

The results are shown in Fig. 1. The high inflammation score seen in the Vehicle administration group of the C-protein-induced mouse myositis model (CIM mouse) was significantly suppressed in the PSL administration group and BCAA administration group. This demonstrates that BCAA has an anti-inflammatory action. Since CIM mouse is considered as a model mouse of polymyositis, BCAA was suggested to be useful for the treatment of polymyositis and the like.

### Example 2 Muscular atrophy suppressive effect of BCAA in CIM mouse

Quadriceps, flexor muscles and triceps were collected from the CIM mouse and control mouse (Ctrl) after 21 days from immunization, and the muscle weight was measured. The results are shown in Fig. 2. A decrease in the muscle weight was observed in all muscles in the Vehicle administration group of the CIM mouse as compared to the Ctrl group. Although the PSL administration group did not show improvement, the BCAA administration group showed improvement.

### Example 3 Muscle strength improving effect of BCAA in CIM mouse

After 20 days from immunization, the muscle strength of the forelimb of each mouse was measured by a veterinary grip dynamometer MK-380CM/R (Muromachi Kikai Co., Ltd.). The measurement was performed 6 times for each animal, and the average thereof was determined. The results are shown in Fig. 3. When compared to the Ctrl group, the muscle strength decreased in the Vehicle administration group of the CIM mouse group. The improvement effect was weak in the PSL administration group, but improvement nearly to the normal level was found in the BCAA administration group.

### Example 4 Muscle fiber atrophy improving effect of BCAA in CIM mouse

Since steroid myopathy is dominantly developed in the fast muscle fiber (MHC IIB-positive fiber), atrophy of the muscle fiber of the CIM mouse was evaluated by measuring the sectional area of the muscle fiber. The boundary area of the fast muscle fiber and the muscle fiber of the section of the triceps collected 21 days after the immunization was labeled by immune fluorescent staining using anti-MHC IIB antibody and anti-Laminin antibody (Sigma), and the sectional area of the MHC IIB-positive muscle fiber was measured using Image J software (NIH). The distribution of the muscle fiber area is shown in the histograms of Fig. 4. When compared to the Ctrl group, a shift toward the left side was found in the histogram showing the atrophy of the muscle fiber of the Vehicle administration group of the CIM mouse, and the tendency was stronger in the PSL administration group. In contrast, the ratio of the thin muscle fiber decreased and the ratio of the thick muscle fiber increased in the BCAA administration group, and a partial improving effect on the muscle fiber atrophy was obtained.

### Example 5 Dose responsiveness of BCAA to suppression of muscular tissue inflammation

According to the method described in Example 1, 0.25 g/kg BCAA+20 mg/kg PSL, 0.75 g/kg BCAA+20 mg/kg PSL, 2.25 g/kg BCAA+20 mg/kg PSL (each BCAA is a mixture of isoleucine, leucine and valine at a weight ratio of isoleucine:leucine:valine=1:2:1.2), or 20 mg/kg PSL was orally administered consecutively, and they were used as PSL single administration group and PSL+BCAA combined administration group. In addition, 0.5% methylcellulose and 5% gamma cyclodextrin were orally administered instead of BCAA and the like to give Vehicle administration group.

The results obtained by the evaluation method described in Example 1 are shown in Fig. 5. In the BCAA+PSL combined administration group, all BCAA+PSL combined use administration groups with different BCAA dose showed a tendency to suppress inflammation more than the PSL single administration group in the inflammation score.

### Example 6 Dose responsiveness of BCAA to muscular atrophy suppression

Quadriceps, hamstrings and triceps were collected from the CIM mouse and control mouse (Ctrl) of Example 5 after 21 days from the immunization, and the muscle weight was measured. The results are shown in Fig. 6. In the PSL single administration group, the muscle weight did not increase, whereas the muscle weight increased dose-dependently in the BCAA+PSL combined administration group.

### Example 7 Dose responsiveness of BCAA to muscle strength improvement

The muscle strength of the forelimb of each mouse was measured after 20 days from the immunization in Example 5 using veterinary grip dynamometer MK-380CM/R (Muromachi Kikai Co., Ltd.). The measurement was performed 6 times for each animal, and the average thereof was determined. The results are shown in Fig. 7. In the PSL single administration group, the strength of the forelimb muscle did not increase, whereas the strength of the forelimb muscle increased dose-dependently in the BCAA+PSL combined administration group.

### Industrial Applicability

When the branched chain amino acids of isoleucine, leucine and valine are combined, inflammation in the muscular tissue is suppressed, and further, the symptoms of muscular atrophy are suppressed and the muscle strength is improved. Therefore, by administering them to patients with idiopathic inflammatory myopathy, such disease can be effectively treated. Furthermore, although a steroid single agent cannot improve muscle strength, the branched chain amino acids of isoleucine, leucine and valine in combination can improve muscle strength even when combined with a steroid drug. Thus, by administering to patients with idiopathic inflammatory myopathy associated with steroid-induced myopathy, such disease can be treated effectively.

Therefore, the present invention is useful as a medicament for the prophylaxis or treatment of idiopathic inflammatory myopathy or idiopathic inflammatory myopathy associated with steroid-induced myopathy that develops during the course of treatment.

This application is based on patent application No. 2012-023521 filed in Japan, the contents of which are encompassed in full herein.

## Claims

1. A prophylactic or therapeutic agent for idiopathic inflammatory myopathy, comprising isoleucine, leucine and valine as active ingredients.

2. The agent according to claim 1, wherein the idiopathic inflammatory myopathy is selected from the group consisting of polymyositis, dermatomyositis, dermatomyositis without muscular symptom, child dermatomyositis, myositis occurring in association with malignant tumor, myositis occurring in association with other collagen disease, and inclusion body myositis.

3. The agent according to claim 1 or 2, which suppresses inflammation of muscular tissue.

4. The agent according to any one of claims 1 to 3, which improves muscular atrophy and/or muscle weakness.

5. The agent according to any one of claims 1 to 4, wherein the weight ratio of isoleucine, leucine and valine is 1:1 - 3:0.5-2.0.

6. The agent according to any one of claims 1 to 5, whose daily dose for human is 3 - 90 g as a total amount of isoleucine, leucine and valine.

7. A method of preventing or improving idiopathic inflammatory myopathy, comprising administering a composition containing isoleucine, leucine and valine as active ingredients to a subject of administration.

8. The method according to claim 7 for suppressing inflammation of muscular tissue, which comprises administering a composition containing isoleucine, leucine and valine as active ingredients to a subject of administration.

9. The method according to claim 7 for improving muscular atrophy and/or muscle weakness, which comprises administering a composition containing isoleucine, leucine and valine as active ingredients to a subject of administration.

10. The method according to any one of claims 7 to 9, wherein the weight ratio of isoleucine, leucine and valine is 1:1 - 3:0.5 - 2.0.

11. The method according to any one of claims 7 to 10, wherein the daily dose for human is 3 - 90 g as a total amount of isoleucine, leucine and valine.

12. The agent according to any one of claims 1 to 6, wherein the idiopathic inflammatory myopathy is idiopathic inflammatory myopathy associated with steroid-induced myopathy which develops in the course of treatment.

13. The method according to any one of claims 7 to 11, wherein the idiopathic inflammatory myopathy is idiopathic inflammatory myopathy associated with steroid-induced myopathy which develops in the course of treatment.
